# EUROPÄISCHE PATENTSCHRIFT

(11) **EP 1 393 685 B1**
(45) Veröffentlichungstag und Bekanntmachung des Hinweises auf die Patenterteilung: **15.06.2005**
(21) Anmeldenummer: 02405757.2
(22) Anmeldetag: 30.08.2002
(51) Int. Cl.: A61B 17/32

(54) **Skalpellklingenhalter und Skalpell**
Scalpel blade holder and scalpel
Porte-lame de scalpel et scalpel

(43) Veröffentlichungstag der Anmeldung: 03.03.2004
(73) Patentinhaber: SIS AG Surgical Instrument Systems, 2555 Brügg (CH)
(72) Erfinder: Ziemer, Frank, 2555 Brügg b. Biel (CH)
(74) Vertreter: Vogel, Dany

(56) Entgegenhaltungen:
- WO-A-02/49520
- FR-A- 483 593
- US-A- 3 384 386
- US-A- 4 149 811
- US-A- 6 039 053

## Beschreibung

### Technisches Gebiet

Die vorliegende Erfindung betrifft einen Skalpellklingenhalter und ein Skalpell. Die Erfindung betrifft insbesondere einen Skalpellklingenhalter und ein Skalpell, welche Mittel zum Befestigen einer Skalpellklinge und einen Griffbereich aufweisen.

### Stand der Technik

Herkömmliche Skalpellklingenhalter und Skalpelle weisen Mittel zur lösbaren oder festen Befestigung von Skalpellklingen auf und verfügen über einen Griffbereich, der einem Benutzer ermöglicht, das Skalpell respektive den Skalpellklingenhalter, mit den Fingern einer Hand zu halten. Bei der Verwendung von Skalpellen und Skalpellklingenhaltern muss sich der Benutzer mittels eines Blicks vergewissern, dass er das Instrument so in den Fingern hält, dass die Skalpellklinge richtig ausgerichtet ist und die Schneide der Skalpellklinge in die gewünschte Richtung zeigt. Um die Skalpellklinge auszurichten, muss der Benutzer folglich den Blick von der Stelle, wo der chirurgische Eingriff vorgenommen werden soll, lösen, was insbesondere beim Einsatz von Mikroskopen oder Vergrösserungsgläsern umständlich ist und zu unnötigen zeitlichen Verzögerungen und zu Ablenkungen der Aufmerksamkeit führt.

In der Patentschrift FR 483593 wird ein Skalpellklingenhalter beschrieben, der mit einem Vorsprung zur Aufnahme einer Skalpellklinge versehen ist. Der Griffbereich des Skalpellklingenhalters nach FR 483593 weist mehrere Seitenflächen auf, die entlang der Längsachse des Skalpellklingenhalters gerade angeordnet sind. Der Griffbereich ist so ausgestaltet, dass er einen Passgriff zulässt, in welchem der Skalpellklingenhalter respektive die daran befestigte Skalpellklinge bezüglich der haltenden Finger eines Benutzers immer gleich ausgerichtet ist.

In der Patentschrift US 6,039,053 wird ein Griff für Kosmetikapplikatoren beschrieben, der eine Bohrung zur Aufnahme der Kosmetikapplikatoren aufweist. Der Griff nach US 6,039,053 umfasst drei Seitenflächen, die entlang der Längsachse des Griffs gerade angeordnet sind, so dass sich für den Griff ein Querschnitt mit einer im Wesentlichen dreieckförmigen Umhüllenden ergibt. Die drei Seitenflächen sind jeweils identisch ausgeführt und weisen eine Vertiefung auf. Wenn der Griff nach US 6,039,053 von einem Benutzer gehalten wird kann der Griff bezüglich der Finger des Benutzers drei Rotationszustände um die Zentrumsachse des Griffs einnehmen, die für den Benutzer nicht taktil unterscheidbar sind.

### Darstellung der Erfindung

Es ist eine Aufgabe der vorliegenden Erfindung, ein neues Skalpell und einen neuen Skalpellklingenhalter vorzuschlagen, welche nicht die Nachteile des Stands der Technik aufweisen und welche insbesondere ermöglichen, eine Skalpellklinge ohne Blickkontakt auszurichten.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass der Griffbereich des Skalpellklingenhalters drei Seitenflächen umfasst, die entlang der Längsachse des Skalpellklingenhalters gerade angeordnet sind, so dass sich für den Griffbereich ein Querschnitt mit einer im Wesentlichen dreieckförmigen Umhüllenden ergibt, und dass mindestens eine der Seitenflächen mit taktilen Erkennungsmerkmalen versehen ist, welche taktilen Erkennungsmerkmale jeweils als Erhebung auf der mindestens einen der Seitenflächen oder als Vertiefung in der mindestens einen der Seitenflächen ausgebildet sind, so dass die taktilen Erkennungsmerkmale die Ausrichtung des Skalpellklingenhalters hinsichtlich dessen Verdrehung um die Zentrumsachse für den Benutzer über den Tastsinn seiner Finger bestimmbar machen. Die dreieckförmige Umhüllende des Querschnitts des Griffbereichs ermöglicht ein passendes Halten des Skalpellklingenhalters zwischen Mittelfinger, Daumen und Zeigfinger, wobei der Skalpellklingenhalter und somit die daran befestigte Skalpellklinge bezüglich der Finger bloss drei verschiedene Rotationszustände um die Zentrumsachse des Griffbereichs des Skalpellklingenhalters einnehmen kann. Da mindestens eine der drei Seitenflächen des Griffbereichs mit taktilen Erkennungsmerkmalen versehen ist, kann der Benutzer somit mittels seines Tastsinns über seine Finger die Ausrichtung des Skalpellklingenhalters und der daran befestigten Skalpellklinge bestimmen, ohne dafür den Blickkontakt zum Skalpellklingenhalter oder zur Skalpellklinge herstellen zu müssen. Mit anderen Worten, der Benutzer kann ohne Blickkontakt zum Skalpellklingenhalter oder zur Skalpellklinge über den Tastsinn die Ausrichtung der Skalpellklinge hinsichtlich der drei verschiedenen Rotationszustände um die Zentrumsachse des Griffbereichs des Skalpellklingenhalters bestimmen. Die dreieckförmige Umhüllende des Querschnitts des Griffbereichs hat zudem den Vorteil, dass der Skalpellklingenhalter im abgelegten Zustand nicht davon rollt und die daran befestiate Skalpellklinge nicht beschädigt wird.

In einer bevorzugten Ausführungsvariante weist die Umhüllende des Querschnitts des Griffbereichs im Wesentlichen die Form eines Bogendreiecks auf und die Ecken der Umhüllenden des Querschnitts des Griffbereichs sind abgerundet. Die Form des Bogendreiecks ermöglicht einen besseren Griff zwischen Mittelfinger, Daumen und Zeigefinger. Auch die abgerundeten Ecken tragen zu einem besseren Griff bei und verhindern zudem, dass sich der Benutzer an den Ecken verletzt oder Schutzhandschuhe beschädigt.

Vorzugsweise ist mindestens eines der taktilen Erkennungsmerkmale als Erhebung ausgebildet. Erhebungen können über den Tastsinn der Finger besonders leicht wahrgenommen werden und brauchen nicht so gross dimensioniert zu werden, wie taktile Erkennungsmerkmale, die als Vertiefungen ausgebildet sind. Überdies können die taktilen Erkennungsmerkmale, beispielsweise ähnlich der Braille-Blindenschrift, so ausgebildet werden, dass sie einen Identifizierungscode repräsentieren, der den Typ der am Skalpellklingenhalter befestigten Skalpellklinge identifiziert.

In einer bevorzugten Ausführungsvariante ist mindestens eines der taktilen Erkennungsmerkmale als Vertiefung ausgebildet. Insbesondere wenn die Vertiefung gross dimensioniert wird, beispielsweise wenn sie sich über mindestens einen Teil der Länge des Skalpellklingenhalters erstreckt, kann sie zur Einsparung von Herstellungsmaterial dienen und das Gesamtgewicht des Skalpellklingenhalters reduzieren. Zudem können gross dimensionierte Vertiefungen auch bei der Gestaltung und Dimensionierung von Wandstärken mitberücksichtigt werden.

In einer bevorzugten Ausführungsvariante sind mindestens zwei der drei Seitenflächen des Griffbereichs mit jeweils voneinander verschiedenen taktilen Erkennungsmerkmalen versehen. Zum Beispiel ist mindestens eines der taktilen Erkennungsmerkmale als Erhebung ausgebildet und erstreckt sich über zwei Seitenflächen des Griffbereichs, und mindestens eines der taktilen Erkennungsmerkmale ist als Vertiefung ausgebildet und ist auf der verbleibenden dritten Seitenfläche des Griffbereichs angeordnet. Eine derartige Gestaltung mit unterschiedlichen taktilen Erkennungsmerkmalen bringt eine weitere Erleichterung der Positionierung und Ausrichtung des Skalpellklingenhalters und der damit befestigten Skalpellklinge mittels des Tastsinns der Finger. Diejenige Seitenfläche des Griffbereichs, die auf dem Mittelfinger abgestützt werden soll, kann beispielsweise mit einem als Vertiefung ausgestalteten taktilen Erkennungsmerkmal versehen werden, während die beiden anderen, durch den Daumen und den Zeigfinger gehaltenen Seitenflächen des Griffbereichs, mit als Erhebungen ausgestalteten taktilen Erkennungsmerkmalen versehen werden.

In einer bevorzugten Ausführungsvariante sind die Erhebungen als Rippen ausgebildet, die sich quer zur Längsachse des Skalpellklingenhalters erstrecken. Dies hat den zusätzlichen Vorteil, dass die Rippen einen verbesserten Halt in der Richtung der Längsachse des Skalpellklingenhalters bieten und ein Rutschen der Finger in dieser Richtung verhindern, insbesondere wenn diese nass sind oder mit einem nassen Handschuh bedeckt sind.

In einer Ausführungsvariante weist der Skalpellklingenhalter einen Endbereich auf, der sich vom Griffbereich aus verjüngt, und die Mittel zum Befestigen einer Skalpellklinge umfassen eine Bohrung zur Aufnahme der Skalpellklinge, die axial durch den Endbereich verläuft.

In einer Ausführungsvariante verläuft die Bohrungsachse von der Zentrumsachse des Griffbereichs versetzt, parallel zur Zentrumsachse des Griffbereichs. Durch die parallele Versetzung der Bohrungsachse kann die am Skalpellklingenhalter befestigte Skalpellklinge näher zur Seitenfläche versetzt werden, die am Mittelfinger abgestützt wird, was die Skalpellklinge näher zum zu behandelnden Objekt bringt. Die kleinere Distanz der Skalpellklinge zum Behandlungsobjekt verringert die notwendigen Bewegungsdistanzen und verbessert die Arbeitsgenauigkeit, insbesondere beim Einsatz in der Opthalmologie.

Die vorliegende Erfindung bezieht sich auch auf ein Skalpell, das einen oben beschriebenen Skalpellklingenhalter und eine daran befestigte Skalpellklinge umfasst.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt eine Schnittansicht eines Skalpellklingenhalters, welche einen Querschnitt des Griffbereichs in Form eines Dreiecks mit abgerundeten Ecken illustriert.
Figur 2 zeigt eine Schnittansicht des Skalpellklingenhalters, welche einen Querschnitt des Griffbereichs in Form eines Bogendreiecks mit abgerundeten Ecken illustriert.
Figur 3 zeigt eine Schnittansicht des Skalpellklingenhalters, welche einen Querschnitt des Griffbereichs illustriert, der eine Umhüllende in Form eines Bogendreiecks mit abgerundeten Ecken aufweist und mit einer Erhebung und einer Vertiefung versehen ist.
Figur 4 zeigt eine dreidimensionale Ansicht des Skalpells mit einem Skalpellklingenhalter und einer daran befestigten Skalpellklinge.
Figur 5 zeigt einen Längsschnitt des Skalpellklingenhalters mit Erhebungen im Griffbereich und einer Vertiefung über einen Teil der Länge des Skalpellklingenhalters.
Figur 6 zeigt eine Seitenansicht des Skalpellklingenhalters mit Erhebungen im Griffbereich.
Figur 7 zeigt eine Aufsicht des Skalpellklingenhalters mit Erhebungen im Griffbereich.

### Wege zur Ausführung der Erfindung

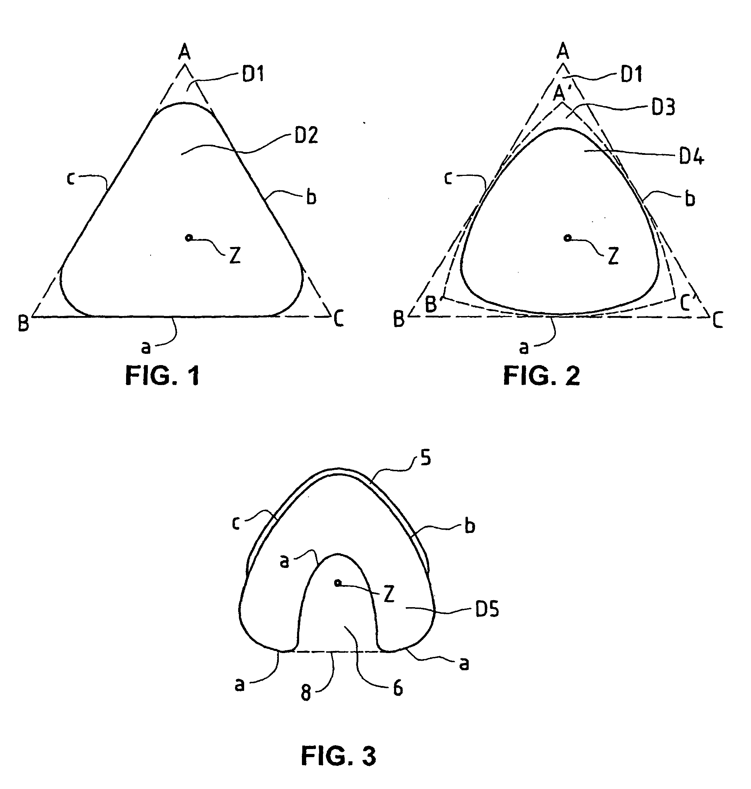

In den Figuren 1 bis 7 werden einander entsprechende gleiche Komponenten durch gleiche Bezugszeichen bezeichnet.

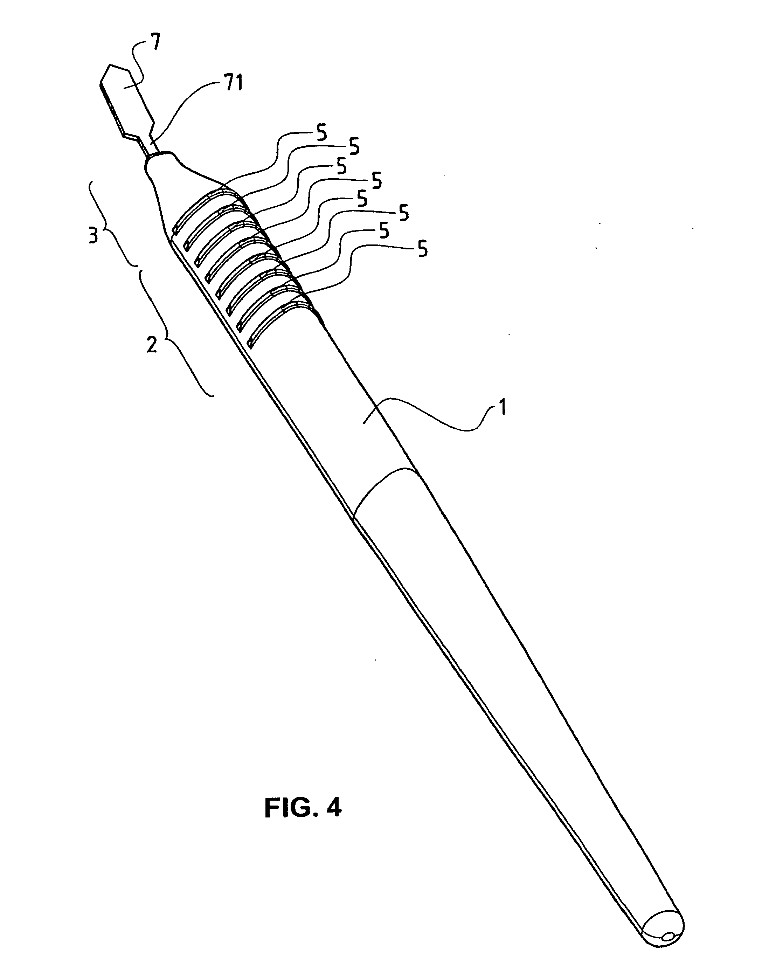

In der Figur 4 wird eine dreidimensionale Ansicht eines Skalpells mit einem Skalpellklingenhalter 1 und einer daran befestigten Skalpellklinge 7 dargestellt. Der Skalpellklingenhalter 1 weist einen Griffbereich 2 und einen Endbereich 3 auf, der sich vom Griffbereich 2 aus verjüngt. Der Griffbereich 2 weist taktile Erkennungsmerkmale in Form von Erhebungen 5 auf.

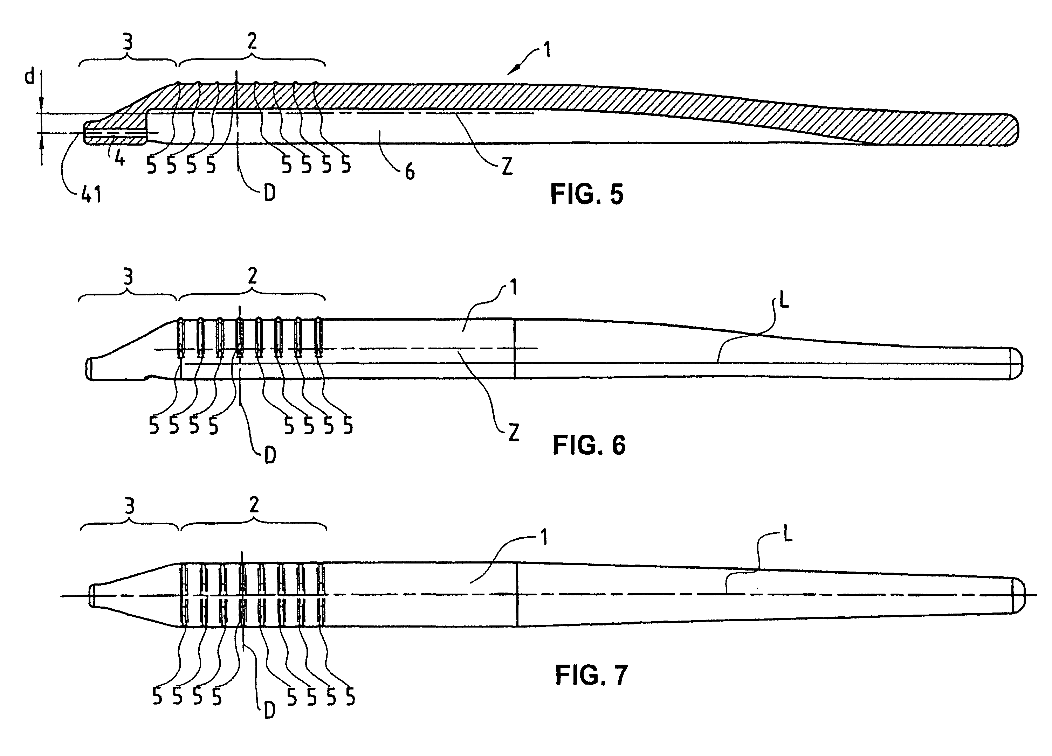

Wie in der Figur 5 dargestellt ist, weist der Endbereich 3 eine Bohrung 4 auf, in welche der Befestigungszylinder 71 der Skalpellklinge 7 (siehe Figur 4) zur Befestigung der Skalpellklinge 7 einführbar ist. Zur Befestigung der Skalpellklinge 7 wird der Befestigungszylinder 71 in der Bohrung 4 verklebt oder verschweisst. Die Befestigung kann auch mittels Presssitz erfolgen. Der Skalpellklingenhalter 1 kann auch im Spritzgussverfahren um den Befestigungszylinder 71 herum gespritzt werden, dabei weist der Befestigungszylinder 71 vorzugsweise an seiner Oberfläche strukturelle Elemente auf.

In den Figuren 1, 2 und 3 sind Schnittansichten des Skalpellklingenhalters 1 dargestellt, welche unterschiedliche mögliche Querschnittsformen D1, D2, D3, D4, D5 des Griffbereichs 2 illustrieren, die alle eine im Wesentlichen dreieckförmige Umhüllende aufweisen. In den Figuren 1, 2 und 3 bezeichnen die Bezugszeichen a, b und c jeweils die Seitenflächen des Griffbereichs 2. Das in den Figuren 1 und 2 dargestellte Dreieck mit den Ecken A, B und C entspricht einem gleichseitigen, dreieckförmigen Querschnitt D1. Gegenüber dem Querschnitt D1 weist der Querschnitt D2 abgerundete Ecken A, B und C auf. Die Umhüllende des Querschnitts D3 entspricht einem Bogendreieck, das sich durch sich in den Schnittpunkten A', B', C' schneidende Kreisbögen ergibt. Gegenüber dem Querschnitt D3 sind die durch die Schnittpunkte A', B' und C' gebildeten Ecken im Querschnitt D4 abgerundet. Der Fachmann wird verstehen, dass das Bogendreieck auch durch andere Bogenformen als Kreisbogen gestaltet werden kann. Zudem können die Bögen auch so angeordnet werden, dass sich eine Querschnittsform mit konkaven Seiten ergibt. Überdies muss die Querschnittsform des Griffbereichs 2 nicht unbedingt symmetrisch zur Zentrumsachse Z sein, das heisst die dreieckförmige Umhüllende muss nicht unbedingt gleichseitig ausgebildet sein und die verschiedenen Ecken müssen nicht alle gleich beschaffen sein. Ausgehend vom Dreieck mit den Ecken A, B und C kann beispielsweise nur eine Ecke A abgerundet oder abgeflacht sein, so dass die im Wesentlichen dreieckförmige Umhüllende einen trapezförmigen Querschnitt umgibt, oder zwei Ecken B, C können stark abgerundet sein während die dritte Ecke A keine Abrundung aufweist, so dass die im Wesentlichen dreieckförmige Umhüllende einen tropfenförmigen Querschnitt umgibt.

Die in der Figur 3 dargestellte Ansicht eines Querschnitts des Griffbereichs 2 entspricht dem in den Figuren 5, 6 und 7 markierten Querschnitt D. In dieser bevorzugten Ausführung des Skalpellklingenhalters 1 weist der Querschnitt D5 des Griffbereichs 2 eine im Wesentlichen dreieckförmige Umhüllende 8 auf, welche die Form eines Bogendreiecks mit abgerundeten Ecken aufweist, die der in der Figur 2 dargestellten Querschnittsform D4 entspricht. Wie in der Figur 3 dargestellt ist, weist der Querschnitt D5 eine Vertiefung 6 auf, die sich durch eine Einbuchtung der Seitenfläche a ergibt.

Wie in der Figur 5 dargestellt ist, ist die Vertiefung 6 als Aussparung gestaltet, erstreckt sich über den gesamten Griffbereich 2 und dehnt sich über den grösseren Teil des Skalpellklingenhalters aus. Wie aus den Figuren 3 und 5 ersichtlich ist, ist die Vertiefung 6 so ausgestaltet, dass sich eine im Wesentlichen gleiche Wandstärke im ganzen Skalpellklingenhalter 1 ergibt, was insbesondere bei der Herstellung des Skalpellklingenhalters 1 mittels Spritzgusstechnik (beispielsweise aus Kunststoff oder Leichtmetall) vorteilhaft ist. Der Fachmann wird verstehen, dass die Vertiefungen 6 auch in anderer Form und insbesondere kleinerer ausgebildet sein können, beispielsweise in kreisförmigen Vertiefungen oder in anderen geometrischen Formen.

In der Figur 3 ist zudem ersichtlich, dass auf dem Querschnitt D5 eine Erhebung 5 angebracht ist, die sich jeweils über einen Teil der Seitenflächen b und c erstreckt. Wie in den Figuren 4, 5, 6 und 7 dargestellt ist, sind mehrere solcher Erhebungen 5 im Griffbereich 2 angeordnet. Die Erhebungen 5 sind in Form von Rippen ausgebildet, die quer zur Längsachse L des Skalpellklingenhalters 1 verlaufen. Der Fachmann wird verstehen, dass die Erhebungen 5 auch in anderer Form ausgebildet sein können, beispielsweise punktförmig wie bei der Braille-Blindenschrift oder in anderen geometrischen Formen.

Die Erhebungen 5 und/oder die Vertiefungen 6 können auch so ausgebildet und/oder angeordnet werden, dass sie einen Code repräsentieren, der dem Benutzer über den Tastsinn Informationen über das von ihm verwendete Skalpell vermittelt, beispielsweise über den Typ der Skalpellklinge 7.

Wie in der Figur 5 dargestellt ist, ist die Bohrungsachse 41 um die Distanz d von der Zentrumsachse Z des Griffbereichs 2 versetzt.

Abschliessend soll hier festgehalten werden, dass sich für den Fachmann weitere Ausführungsformen des Skalpellklingenhalters 1 gemäss den Ansprüchen ergeben. Beispielsweise können die Seitenflächen a, b, c des Griffbereichs 2 mit unterschiedlichen, seitenspezifischen taktilen Merkmalen versehen werden, die sich in ihrer geometrischen Ausgestaltung und/oder Anordnung unterscheiden. Zudem soll hier der Klarheit halber angefügt werden, dass an Stelle einer Skalpellklinge 7 mit einer Schneidefläche auch andere Instrumente am Skalpellklingenhalter 1 befestigt werden können die nicht unbedingt eine Schneidefläche aufzuweisen brauchen.

## Patentansprüche

1. Skalpellklingenhalter (1 ) mit Mitteln zum Befestigen einer Skalpellklinge (7) und mit einem Griffbereich (2), wobei der Griffbereich (2) drei Seitenflächen (a, b, c) umfasst, die entlang der Längsachse (L) des Skalpellklingenhalters (1) gerade angeordnet sind, so dass sich für den Griffbereich (2) ein Querschnitt (D5) mit einer im Wesentlichen dreieckförmigen Umhüllenden (8) ergibt, **dadurch gekennzeichnet,**
**dass** die drei Seitenflächen (a, b, c) so angeordnet sind, dass bei passendem halten des Griffbereichs (2) zwischen Mittelfinger, Daumen und Zeigefinger eines Benutzers vom Skalpellklingenhalter (1) bezüglich der Finger nur drei verschiedene Rotationszustände um die Zentrumsachse (Z) des Griffbereichs (2) einnehmbar sind, und
**dass** mindestens eine der Seitenflächen (a, b, c) mit taktilen Erkennungsmerkmalen versehen ist, welche taktilen Erkennungsmerkmale jeweils als Erhebung (5) auf der mindestens einen der Seitenflächen (a, b, c) oder als Vertiefung (6) in der mindestens einen der Seitenflächen (a, b, c) ausgebildet sind, so dass die taktilen Erkennungsmerkmale die Ausrichtung des Skalpellklingenhalters (1) hinsichtlich der drei verschiedenen Rotationszustände um die Zentrumsachse (Z) für den Benutzer über den Tastsinn seiner Finger bestimmbar machen.

2. Skalpellklingenhalter (1) nach Anspruch 1, **dadurch gekennzeichnet, dass** sich die Vertiefung (6) über mindestens einen Teil der Länge des Skalpellklingenhalters (1 ) erstreckt.

3. Skalpellklingenhalter (1) nach einem der Ansprüche 1 oder 2, **dadurch gekennzeichnet, dass** mindestens zwei der Seitenflächen (a, b, c) mit jeweils voneinander verschiedenen taktilen Erkennungsmerkmalen versehen sind.

4. Skalpellklingenhalter (1) nach Anspruch 1, **dadurch gekennzeichnet, dass** mindestens eines der taktilen Erkennungsmerkmale als Erhebung (5) ausgebildet ist und sich über zwei der Seitenflächen (b, c) erstreckt und dass mindestens eines der taktilen Erkennungsmerkmale als Vertiefung (6) ausgebildet ist und auf der verbleibenden dritten Seitenfläche (a) angeordnet ist.

5. Skalpellklingenhalter (1) nach einem der Ansprüche 1 bis 4, **dadurch gekennzeichnet, dass** die Erhebungen (5) als Rippen ausgebildet sind, die sich quer zur Längsachse (L) des Skalpellklingenhalters (1 ) erstrecken.

6. Skalpellklingenhalter (1) nach einem der Ansprüche 1 bis 5, **dadurch gekennzeichnet, dass** die Umhüllende (8) des Querschnitts des Griffbereichs (2) im Wesentlichen die Form eines Bogendreiecks aufweist und dass die Umhüllende (8) des Querschnitts (D5) des Griffbereichs (2) abgerundete Ecken aufweist.

7. Skalpellklingenhalter (1) nach einem der Ansprüche 1 bis 6, **dadurch gekennzeichnet, dass** er einen Endbereich (3) aufweist, der sich vom Griffbereich (2) aus verjüngt und dass die Mittel zum Befestigen einer Skalpellklinge (7) eine Bohrung (4) zur Aufnahme der Skalpellklinge (7) umfassen, die axial durch den Endbereich (3) verläuft.

8. Skalpellklingenhalter (1) nach Anspruch 7, **dadurch gekennzeichnet, dass** die Bohrungsachse (41 ) der Bohrung (4) von der Zentrumsachse (Z) des Griffbereichs (2) versetzt, parallel zur Zentrumsachse (Z) des Griffbereichs (2) verläuft.

9. Skalpell, das einen Skalpellklingenhalter (1) nach einem der Ansprüche 1 bis 8 und eine daran befestigte Skalpellklinge (7) umfasst.

10. Skalpell nach Anspruch 9, **dadurch gekennzeichnet, dass** die taktilen Erkennungsmerkmale so ausgeführt sind, dass sie einen Identifizierungscode repräsentieren, der den Typ der Skalpellklinge (7) identifiziert.

## Claims

1. Scalpel blade holder (1) with means for securing a scalpel blade (7) and with a grip area (2), the grip area (2) comprising three side faces (a, b, c) which are arranged rectilinearly along the longitudinal axis (L) of the scalpel blade holder (1), so that a cross section (D5) with a substantially triangular envelope (8) is obtained for the grip area (2), **characterized**
**in that** the three side faces (a, b, c) are arranged in such a way that, when the grip area (2) is held correctly between the middle finger, thumb and index finger of a person using the scalpel blade holder (1), only three different states of rotation about the centre axis (Z) of the grip area (2) can be adopted relative to the fingers, and
**in that** at least one of the side faces (a, b, c) is provided with tactile identification features, which tactile identification features are designed in each case as an elevation (5) on the at least one of the side faces (a, b, c) or as a depression (6) in the at least one of the side faces (a, b, c), the tactile identification features allowing the user to determine, using the sense of touch in his fingers, the orientation of the scalpel blade holder (1) in relation to the three different states of rotation about the centre axis (Z).

2. Scalpel blade holder (1) according to Claim 1, **characterized in that** the depression (6) extends along at least part of the length of the scalpel blade holder (1).

3. Scalpel blade holder (1) according to either of Claims 1 and 2, **characterized in that** at least two of the side faces (a, b, c) are provided with tactile identification features differing from each other.

4. Scalpel blade holder (1) according to Claim 1, **characterized in that** at least one of the tactile identification features is designed as an elevation (5) and extends over two of the side faces (b, c), and **in that** at least one of the tactile identification features is designed as a depression (6) and is arranged on the remaining third side face (a).

5. Scalpel blade holder (1) according to one of Claims 1 to 4, **characterized in that** the elevations (5) are designed as ribs which extend transversely with respect to the longitudinal axis (L) of the scalpel blade holder (1).

6. Scalpel blade holder (1) according to one of Claims 1 to 5, **characterized in that** the envelope (8) of the cross section of the grip area (2) has substantially the shape of a curved triangle, and
**in that** the envelope (8) of the cross section (D5) of the grip area (2) has rounded corners.

7. Scalpel blade holder (1) according to one of Claims 1 to 6, **characterized in that** it has an end area (3) which tapers from the grip area (2), and
**in that** the means for securing a scalpel blade (7) comprise a bore (4) for receiving the scalpel blade (7), which bore extends axially through the end area (3).

8. Scalpel blade holder (1) according to Claim 7, **characterized in that** the bore axis (41) of the bore (4) is offset from the centre axis (Z) of the grip area (2) and extends parallel to the centre axis (Z) of the grip area (2).

9. Scalpel which comprises a scalpel blade holder (1) according to one of Claims 1 to 8 and a scalpel blade (7) secured thereon.

10. Scalpel according to Claim 9, **characterized in that** the tactile identification features are designed so that they represent an identification code which identifies the type of scalpel blade (7).

## Revendications

1. Porte-lame de scalpel (1) comprenant des moyens pour la fixation d'une lame de scalpel (7) et une zone de préhension (2), la zone de préhension (2) comprenant trois faces latérales (a, b, c) qui sont disposées directement le long de l'axe longitudinal (L) du porte-lame de scalpel (1), de sorte que l'on obtienne une section transversale (D5) pour la zone de préhension (2) avec une extrémité d'enveloppement (8) de forme essentiellement triangulaire, **caractérisé en ce que**
les trois faces latérales (a, b, c) sont disposées de telle sorte que lors du maintien ajusté de la zone de préhension (2) entre le majeur, le pouce et l'index d'un utilisateur, seulement trois états de rotation différents autour de l'axe central (Z) de la zone de préhension (2) puissent être adoptés par le porte-lame de scalpel par rapport aux doigts, et
**en ce qu'**au moins l'une des faces latérales (a, b, c) est pourvue de marques de reconnaissance tactiles, lesquelles marques de reconnaissance tactiles sont réalisées à chaque fois sous forme de rehaussement (5) sur l'au moins une des faces latérales (a, b, c) ou sous forme de renfoncement (6) dans l'au moins une des faces latérales (a, b, c), de sorte que les marques de reconnaissance tactiles permettent à l'utilisateur de déterminer l'orientation du porte-lame de scalpel (1) par rapport aux trois états de rotation différents autour de l'axe central (Z), par le biais du sens tactile de ses doigts.

2. Porte-lame de scalpel (1) selon la revendication 1, **caractérisé en ce que** le renfoncement (6) s'étend sur au moins une partie de la longueur du porte-lame de scalpel (1).

3. Porte-lame de scalpel (1) selon l'une quelconque des revendications 1 ou 2, **caractérisé en ce qu'**au moins deux des faces latérales (a, b, c) sont pourvues à chaque fois de marques de reconnaissance tactiles différentes les unes des autres.

4. Porte-lame de scalpel (1) selon la revendication 1, **caractérisé en ce qu'**au moins l'une des marques de reconnaissance tactiles est réalisée sous forme de rehaussement (5) et s'étend sur deux des faces latérales (b, c) et **en ce qu'**au moins l'une des marques de reconnaissance tactiles est réalisée sous forme de renfoncement (6) et est disposée sur la troisième face latérale (a) restante.

5. Porte-lame de scalpel (1) selon l'une quelconque des revendications 1 à 4, **caractérisé en ce que** les rehaussements (5) sont réalisés sous forme de nervures qui s'étendent transversalement à l'axe longitudinal (L) du porte-lame de scalpel (1).

6. Porte-lame de scalpel (1) selon l'une quelconque des revendications 1 à 5, **caractérisé en ce que** l'extrémité d'enveloppement (8) de la section transversale de la zone de préhension (2) présente essentiellement la forme d'un triangle cintrée et **en ce que** l'extrémité d'enveloppement (8) de la section transversale (D5) de la zone de préhension (2) présente des coins arrondis.

7. Porte-lame de scalpel (1) selon l'une quelconque des revendications 1 à 6, **caractérisé en ce qu'**il présente une zone d'extrémité (3) qui se rétrécit depuis la zone de préhension (2) et **en ce que** les moyens pour la fixation d'une lame de scalpel (7) comprennent un alésage (4) pour recevoir la lame de scalpel (7), qui s'étend axialement à travers la zone d'extrémité (3).

8. Porte-lame de scalpel (1) selon la revendication 7, **caractérisé en ce que** l'axe (41) de l'alésage (4) s'étend de manière décalée de l'axe central (Z) de la zone de préhension (2), parallèlement à l'axe central (Z) de la zone de préhension (2).

9. Scalpel, qui comprend un porte-lame de scalpel (1) selon l'une quelconque des revendications 1 à 8, et une lame de scalpel (7) fixée sur celui-ci.

10. Scalpel selon la revendication 9, **caractérisé en ce que** les marques de reconnaissance tactiles sont réalisées de telle sorte qu'elles représentent un code d'identification qui identifie le type de lame de scalpel (7).
